(19) Europäisches Patentamt
European Patent Office
Office européen des brevets

(11) Veröffentlichungsnummer: **0 220 676**
**A1**

# (12) EUROPÄISCHE PATENTANMELDUNG

(21) Anmeldenummer: **86114683.5**

(22) Anmeldetag: **22.10.86**

(51) Int. Cl.⁴: **C07C 103/38**, C07H 15/04, A61K 7/00, A61K 47/00, C11D 1/52

(30) Priorität: **29.10.85 DE 3538451**

(43) Veröffentlichungstag der Anmeldung:
**06.05.87 Patentblatt 87/19**

(84) Benannte Vertragsstaaten:
**AT BE CH DE FR GB IT LI NL SE**

(71) Anmelder: **Süddeutsche Zucker-Aktiengesellschaft**
**Maximilianstrasse 10**
**D-6800 Mannheim 1(DE)**

(72) Erfinder: **Kunz, Markwart, Dr.**
**Kötherberg 6**
**D-3300 Braunschweig(DE)**

(74) Vertreter: **Körber, Wolfhart, Dr.**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**D-8000 München 22(DE)**

## (54) Fettsäureamide von Aminopolyolen als nichtionogene Tenside.

(57) Die vorliegende Erfindung betrifft neue Tenside aus Fettsäureamiden von Aminopolyolen, entsprechend der allgemeinen Formel 1, ihre Herstellung durch reduktive Aminierung aus Mono-, Di-oder Oligosaccharidaldosen und ihre Verwendung.

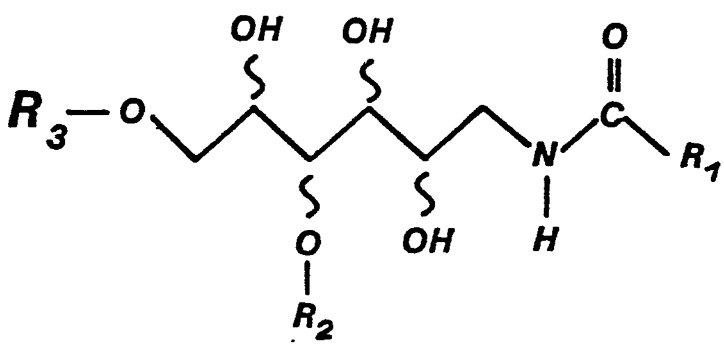

EP 0 220 676 A1

$R_1$ = Alkylgruppe ($C_7$ -$C_{21}$)

$R_2$ = H-oder ein $\alpha$-bzw. $\beta$-glykosidisch verknüpftes Mono-oder Oligosaccharid

$R_3$ = H-oder ein $\alpha$-bzw. $\beta$-glykosidisch verknüpftes Mono-oder Oligosaccharid

## Fettsäureamide von Aminopolyolen als nichtionogene Tenside

Die vorliegende Erfindung betrifft neue Tenside aus Fettsäureamiden von Aminopolyolen, entsprechend der allgemeinen Formel 1, ihre Herstellung durch reduktive Aminierung aus Mono-, Di-oder Oligosaccharidaldosen und ihre Verwendung.

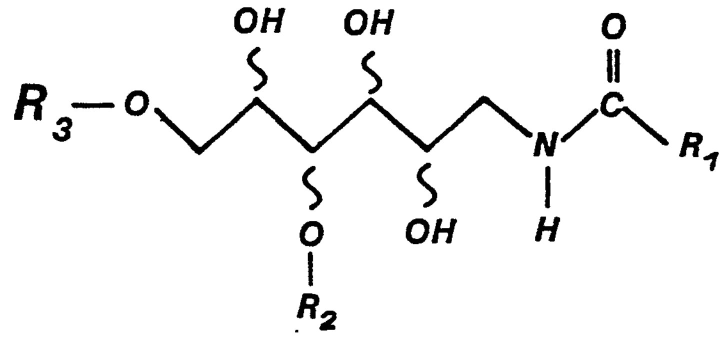

$R_1$ = Alkylgruppe ($C_7$ -$C_{21}$)

$R_2$ = H-oder ein α-bzw. β-glykosidisch verknüpftes Mono-oder Oligosaccharid

$R_3$ = H-oder ein α-bzw. β-glykosidisch verknüpftes Mono-oder Oligosaccharid

Fettsäureamide von Aminopolyolen, hergestellt aus Kohlenhydraten, sind eine neue Klasse nichtionogener Tenside. Andere nichtionogene Kohlenhydrattenside sind in der Literatur vielfach beschrieben, siehe Ullmanns Encyklopädie der Technischen Chemie, 4. Auflage, Bd. 22, 1982, S. 494 ff; Emulgatoren für Lebensmittel, Hrsg. G. Schuster, Springer Verlag Berlin Heidelberg N. Y. 1985; Sucrochemistry, Hrsg. J. L. Hickson, Washington 1977 (ACS Symposium Series Nr. 41).

Vorwiegend handelt es sich dabei um Fettsäureester von Zuckeralkoholen, z. B. Sorbit, oder um Ester nichtreduzierender Disaccharide, wie Saccharose. Zur Erhöhung der Wasserlöslichkeit der Ester von Monosacchariden, wie Sorbit bzw. Sorbitan, wird häufig eine Ethoxylierung durchgeführt; diese Behandlung ist bei Oligosaccharidmonoestern im allgemeinen wegen der besseren Wasserlöslichkeit nicht erforderlich. Besonders bei der Produktion von Oligosaccharidtensiden entsteht im allgemeinen ein Gemisch aus unterschiedlichen Anteilen an Mono-und höher veresterten Produkten, wodurch ihre Anwendungsmöglichkeiten wegen Uneinheitlich keit eingeschränkt werden. Auch die relative geringe Stabilität der Esterbindung dieser Produkte im schwach Sauren und im schwach Alkalischen begrenzt die Einsatzmöglichkeiten.

In den Patenten DE 1 155 771 und US 2 662 073 sind Amide auf Kohlenhydratbasis bereits beschrieben worden. Bei diesen Verbindungen handelt es sich jedoch um Amide von Polyhydroxy karbonsäuren mit Fettaminen, während die erfindungsgemäßen Verbindungen Amide von Fettsäuren mit Aminopolyolen sind.

Die Herstellung der in den Patenten erwähnten Polyhydroxykarbonsäureamide erfolgt unter Verwendung von Fettaminen in Lösungsmitteln, wie Dimethylformamid, die sich nicht ohne weiteres wieder rückstandslos aus den Produkten entfernen lassen. Solche Rückstände schränken die Anwendungsmöglichkeiten dieser Produkten besonders in Lebensmitteln ein. Bei einem wechselseitigen Austausch der reagierenden Gruppen ändern sich häufig auch die Eigenschaften der jeweils resultierenden Verbindungen, beispielsweise haben Ameisen säureethylester und Essigsäuremethylester unterschiedliche Schmelz-und Siedepunkte.

Die erfindungsgemäßen Verbindungen haben gegenüber den zuvor erwähnten nichtionogenen Tensiden aufgrund ihrer Struktur und Herstellung vor allem im im anwendungstechnischen Bereich Vorteile. Ebenso sind sie flüssigkristallin und eignen sich zur Herstellung von Membranen.

Wie bereits erwähnt, sind Ester in wäßrigen Lösungen hydrolyseempfindlich. So stellt z. B. Schuster fest: "Die allgemeinen Eigenschaften der Zuckerester sind dadurch geprägt, daß sie Fettsäurester eines mehrwertigen Alkohols sind. Dadurch zeigen sie Umlagerungsreaktionen und Hydrolyseempfindlichkeit". Die meisten Reinigungsvorgänge im Haushalt, wie z. B. maschinelles Wäschewaschen und Geschirrspülen, finden unter alkalischen Bedingungen statt.

Die erfindungsgemäßen Verbindungen wurden im alkalischen Bereich auf ihre Stabilität geprüft. So konnten beispielsweise bei dem Stearylamid von Maltamin auch nach 24 Stunden unter alkalischen Bedingungen (pH 9) bei 60°C dünnschichtchromatographisch keine Hydrolyseprodukte nachgewiesen werden. Daher sind die erfindungsgemäßen Verbindungen in Tensidformulierungen für Reinigungszwecke, beispielsweise von Textilien oder Geschirr, besser geeignet als herkömmliche, nichtionogene Kohlenhydrattenside.

Kosmetische Präparate haben häufig einen schwach sauren pH-Wert und müssen auch bei längerer Lagerung stabil bleiben. Unter ähnlichen Bedingungen wurden die erfindungsgemäßen Verbindungen untersucht. So konnte beispielsweise beim Stearylamid von Maltamin bei einem pH-Wert von ca. 5 und einer Temperatur von 60°C auch nach 24 Stunden dünnschichtchromatographisch kein Hydrolyseprodukt nachgewiesen werden. Daher eignen sich die erfindungsgemäßen Verbindungen besser für kosmetische Präparate, als herkömmliche vergleichbare Ester.

In Nahrungsmitteln und Pharmazeutika sollen heute nach Möglichkeit chemisch einheitliche Produkte als Zusatzstoffe eingesetzt werden. So hat sich z. B. ein Gemisch von Zuckerestern, Triglyceriden, Zucker- und Kaliumseifen, hergestellt nach dem TAL-Verfahren,GB-PS139 9053, in der Praxis nicht durchgesetzt. Wie bereits erläutert, sind die in der Lebensmitteltechnik z. Z. eingesetzten Polyolester, insbesondere die Saccharoseester, Gemische aus unterschiedlichen Anteilen von Mono-und höher veresterten Verbindungen. Wie Schuster feststellt, "bestehen die Monoesterfraktionen der Saccharoseester selbst nach Reinigung aus mindestens 3 stellungsisomeren Estern".

Im Gegensatz dazu handelt es sich bei den erfindungsgemäßen Verbindungen um chemisch einheitliche Monoamide. Ferner enthalten sie aufgrund ihrer Herstellung (s. Beispiele) keine Rückstände von toxikologisch problematischen Substanzen, wie Dimethylformamid oder auch Fettaminen. Daher sind die erfindungsgemäßen Verbindungen als Lebensmittelzusatzstoffe besser geeignet als die zuvor erwähnten nichtionogenen Tenside auf Kohlenhydratbasis.

Darstellung der erfindungsgemäßen Verbindungen

Beispiel

a) Reduktive Aminierung der Kohlenhydrate

Reduktive Aminierung von Mono-und Oligosacchariden sind in der Literatur mehrfach beschrieben. Zum einen erfolgt die Reduktion in Gegenwart von Ammoniak, z. B. nach dem US-Patent 2.016.962, zum anderen in Gegenwart von Hydrazin, z. B. dem US-Patent 2.830.983. Für die Darstellung der erfindungsgemäßen Verbindungen können beide Methoden benutzt werden, vorgezogen wird wegen der höheren Ausbeute die Reduktion in Gegenwart von Hydrazin. Nach der Reduktion erhält man als Rohprodukte beispielsweise aus Glucose 1-Aminosorbit (Glucamin) in ca. 80%iger Ausbeute bzw. aus Maltose 1-Aminomaltit (Maltamin) in ca. 90%iger Ausbeute. Die Rohprodukte können nach der üblichen Aufarbeitung - (Filtration, Destillation zur Trockne) unmittelbar zur weiteren Reaktion verwendet werden.

b) Amidierung der dargestellten Aminopolyole

Die gut getrockneten Amine werden in absolutem Methanol suspendiert und bei Raumtemperatur mit equimolarer oder überstöchiometrischer Menge eines Fettsäureanhydrides versetzt. Vorzugsweise findet die Amidierung in Gegenwart eines basischen Katalysators, wie Kaliumcarbonat, Natriumcarbonat oder Trimethylamin, insbesondere aber in Gegenwart eines basischen Ionenaustauschers statt, um Konkurrenzreaktionen, wie Verersterung zu vermeiden. Nach ca. 16 Stunden Reaktionszeit wird das Rohprodukt in einer Soxhlettapparatur mit Ether, gegebenenfalls auch mit mit einem anderen, unpolarem Lösungsmittel, wie z. B. Essigester, Chloroform oder Toluol, extrahiert. Der verbliebene Rückstand wird in Butanol aufgenommen, filtriert und die Butanollösung mit einer ca. 5%igen wäßrigen Natriumchloridlösung mehrfach ausgeschüttelt. Die Butanolphase wird mit einem Trocknungsmittel, wie Magnesiumsulfat, Natriumsulfat oder Kaliumcarbonat, getrocknet und anschließend das Butanol abdestilliert. Im erhaltenen Rückstand liegen in ca. 60%iger Ausbeute an die erfindungsgemäßen Verbindungen in reiner Form vor.

**Ansprüche**

1. Fettsäureamide der allgemeinen Formel (1) mit einer C-Kettenlänge im Alkylteil von $C_7$ -$C_{21}$, von durch reduktive Aminierung aus Mono-, Di-oder Oligosaccharidaldosen gewonnenen Aminopolyolen.

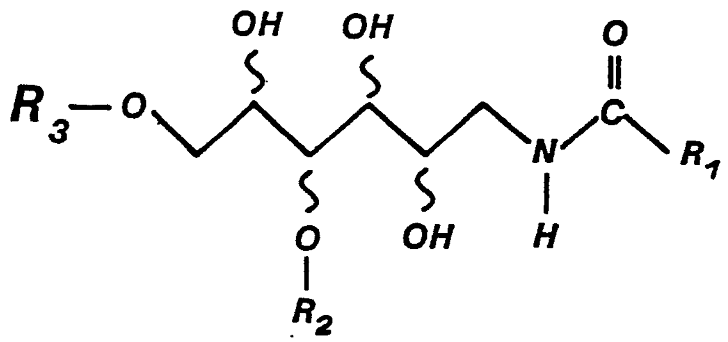

(1)

$R_1$ = Alkylgruppe ($C_7$ -$C_{21}$)

$R_2$ = H-oder ein $\alpha$-bzw. $\beta$-glykosidisch verknüpftes Mono-oder Oligosaccharid

$R_3$ = H-oder ein $\alpha$-bzw. $\beta$-glykosidisch verknüpftes Mono-oder Oligosaccharid

2. Verfahren zur Herstellung der Verbindungen gemäß Anspruch 1, dadurch gekennzeichnet, daß Kohlenhydrate, wie beispielsweise Glucose, Maltose, Laktose, Cellobiose oder höhere Oligosaccharide, durch reduktive Aminierung in an sich bekannter Weise in Aminopolyole überführt werden, diese dann in einem Lösungsmittel, wie Wasser, Methanol oder anderen niederen Alkoholen bzw. Mischungen der Lösungsmittel untereinander, mit einem Fettsäureanhydrid oder Fettsäurehalogenid, vorzugsweise in Gegenwart einer basischen Substanz, wie Kaliumcarbonat, Methylamin oder eines basischen Ionenaustauschers, bei Temperaturen zwischen -10 bis +100 °C, vorzugsweise zwischen 0 und 50 °C N-acyliert werden, und die so erhaltenen Produkte durch Extraktion nicht umgesetzter Edukte, zum einen durch Fest-Flüssigextraktion mit einem unpolaren organischen Lösungsmittel, wie z.B. Ether, Chloroform, Essigester oder Toluol,zum anderen durch Flüssig/Flüssigextraktion, einer mit Wasser nicht mischbaren alkoholischen Lösung, vorzugsweise Butanol mit einer kochsalzhaltigen wässrigen Lösung gereinigt werden.

3. Verwendung der Verbindungen gemäß Anspruch 1 als amphiphile Substanzen

für Reinigungszwecke im allgemeinen,

für kosmetsiche Zwecke, auch als Hilfsstoff zur Herstellung von kosmetischen Präparaten,

bei der Lebensmittelherstellung sowie für pharmazeutische Zwecke, auch als Hilfsstoff zur Herstellung von pharmazeutischen Präparaten.

Europäisches Patentamt

# EUROPÄISCHER RECHERCHENBERICHT

Nummer der Anmeldung

EP 86 11 4683

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-1 360 018 (COMMERCIAL SOLVENTS CORPORATION)<br>* Zusammenfassung *<br>--- | 1,3 | C 07 C 103/38<br>C 07 H 15/04<br>A 61 K 7/00<br>A 61 K 47/10<br>C 11 D 1/52 |
| Y | US-A-2 703 798 (A.M. SCHWARTZ)<br>* Ansprüche *<br>--- | 1,3 | |
| Y | US-A-2 752 334 (H. WACTON)<br>* Ansprüche *<br>--- | 1,3 | |
| Y | DE-B-1 155 771 (DEUTSCHE AKADEMIE DER WISSENSCHAFTEN ZU BERLIN)<br>* Ansprüche *<br>--- | 1,3 | |
| Y | GB-A- 771 423 (ROHM & HAAS)<br>* Ansprüche *<br>--- | 1,3 | RECHERCHIERTE SACHGEBIETE (Int. Cl.4) |
| Y | FR-A-1 509 917 (UPJOHN)<br>* Zusammenfassung *<br>--- | 1,3 | C 07 C 103/00<br>C 07 H 15/00<br>A 61 K 7/00<br>A 61 K 47/00<br>C 11 D 1/00 |
| Y | WO-A-8 304 412 (NATIONAL RESEARCH DEVELOPMENT CORPORATION)<br>* Ansprüche *<br>----- | 1-3 | |

Der vorliegende Recherchenbericht wurde für alle Patentansprüche erstellt.

| Recherchenort | Abschlußdatum der Recherche | Prüfer |
|---|---|---|
| DEN HAAG | 04-02-1987 | MOREAU J.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument